# EUROPEAN PATENT SPECIFICATION

(11) **EP 2 959 919 B1**
(45) Date of publication and mention of the grant of the patent: **12.09.2018**
(21) Application number: 14306004.4
(22) Date of filing: 25.06.2014
(51) Int. Cl.: A61L 2/07

(54) **Apparatus and method for the preparation and sterilization of viscous products containing temperature sensitive compounds**
Vorrichtung und Verfahren zur Herstellung und Sterilisation dickflüssiger Produkte mit temperaturempfindlichen Verbindungen
Appareil et procédé pour la préparation et la stérilisation de produits visqueux contenant des composés sensibles à la température

(43) Date of publication of application: 30.12.2015
(73) Proprietor: SPX APV Danmark A/S, 8600 Silkeborg (DK)
(72) Inventor: Coelho, Gil, 27110 EPREVILLE PRES LE NEUBOURG (FR); Mares, Philippe, 27006 EVREUX (FR)
(74) Representative: Collet, Alain

(56) References cited:
- EP-A2- 1 734 073
- WO-A2-02/072156
- WO-A2-2007/148022
- JP-A- H09 175 985
- US-A1- 2014 165 634

## Description

### TECHNICAL FIELD

The present invention relates to apparatuses and methods for preparing and sterilizing a viscous fluid containing a temperature sensitive compound diluted in a viscous matrix. Such viscous fluid includes, without limitation, cosmetic products, drugs, vegetal and animal extracts, and/or food products into which is diluted a temperature sensitive compound such as for example a perfume or a pharmaceutical active compound. The present invention is particularly suited to the preparation and sterilization of cosmetic cream containing temperature-sensitive perfumes. The invention relates more generally to devices, systems, and methods for processing viscous fluids.

### BACKGROUND ART

The industrial production of food has faced for years the issue of aseptic production and packaging. The aim of such production and packaging is to extend shelf life of products, to preserve nutritional qualities for an extended period of time and to kill germs potentially harmful to health. Government regulations have rapidly constrained the range of options available to extend product shelf life and specifically banned inclusion of many preservatives in foodstuffs destined for human ingestion.

Heat treatment of liquid food products for the purpose of sterilization is a commonly used industrial process. To achieve rapid heating up to high temperatures, typically exceeding 100°C, steam is employed. The heating may take place either directly or indirectly. In indirect heating, use is made of different types of heat exchangers. In direct heating, steam is directly incorporated into the product. A sterilization process, well known for dairy products, is commonly referred to as the UHT process, which stands for *Ultra Heat Treatment* or *Ultra High Temperature;* an appropriate control of both temperature level and exposure duration of the heat treatment allowing the extermination of those microorganisms which are found in dairy products.

There are two types of steam incorporation processes: injection and infusion. With injection, steam is injected into the product in a closed system, whereas infusion implies that the product is finely sprayed and caused to pass through a space filled with steam. In both cases, the supplied steam rapidly and efficiently heats the product to the desired temperature and the product is then kept at this temperature during a given predetermined exposure duration. The supplied steam must thereafter be removed from the product in order to avoid diluting it.

This normally takes place by evaporative cooling, commonly referred as flash-cooling, in a vacuum chamber. During the process, the steam is released and condensed at the same time as the product is cooled down to a similar temperature it had before the heat treatment. The evaporative cooling usually takes place by feeding the steamed product, under pressure, into a vacuum chamber. When the product enters the vacuum chamber, the liquid boils, the steam is released and rises upwards in the chamber while the product accumulates in the lower region of the chamber. Thus cooled, the product may be tapped off from the lower region of the chamber. The steam which leaves the product together with incondensable gases is to be condensed in order for it to be able to be run off to an outlet.

The cosmetic industry is facing a similar issue. As one example, skincare and cosmetic creams are meant to be used multiple times, for a use life extended to months. To address that issue, the cosmetic industry includes various preservatives, such as parabens, which prevent the growth of fungi and bacteria. A number of studies highlighted the potential harmfulness of such preservatives to human health. Other compounds have been investigated as paraben substitutes, but show lower efficiency and/or still carry the risk of long term impact on human health. Consequently, regulations are being more and more restrictive about preservatives integration into cosmetic products.

As an alternative route to incorporating preservatives, it was thought to sterilize cosmetic products. The high viscosity of the products prohibits the use of filters. Several methods and devices based upon heating were investigated. In particular, United States Patent Application US 2009/0148343 discloses the sterilization of a cosmetic product based upon indirect heating, by means of circulating the product through a circuit traversing heating and cooling baths.

JP9175985 shows a method to sterilize additive-free cosmetics. Heat sensitive compounds are subjected to sterile filtration at low temperatures. Other compounds of the composition are heat sterilized and then mixed with the sterilized heat sensitive compounds.

To improve the efficiency of the sterilization process, apparatus and method to sterilize viscous products based upon the alternative direct heating have been developed. However, it remains to be found a sterilizing method that allows keeping the expected properties of a temperature sensitive active compound diluted into a viscous matrix.

### SUMMARY OF THE INVENTION

The sterilization of cosmetic products is a good solution to extend shelf life and preserve properties of the product for an extended period of time while avoiding the inclusion of various additives potentially harmful to health. One challenge is to eliminate or at least minimize the denaturization - caused by heat treatment - of temperature sensitive compound contained in such cosmetic products.

It is one object of the present invention to cope with the disadvantages of the state of the art by providing an apparatus and method for the preparation and sterilization of a viscous fluid containing at least one temperature sensitive compound diluted in a viscous matrix.

To this end, certain embodiments of the present invention relate to an apparatus for the preparation and sterilization of a viscous fluid containing a sterile temperature sensitive compound diluted into a viscous matrix, comprising:
- a heat treatment unit, fed with the viscous matrix, wherein the viscous matrix is sterilized,
- a sterilizing device (27), fed with a temperature sensitive compound (11), ensuring the sterilization of the temperature sensitive compound (11),
- a mixing unit, fed with the sterilized viscous matrix and the sterile temperature sensitive compound, wherein the temperature sensitive compound is incorporated into the sterilized viscous matrix to form the viscous fluid, characterized in that it further comprises:
   - a dosing pump (26) located upstream the sterilizing device (27), fed with the temperature sensitive compound (11) and configured to dose the flow rate or quantity of temperature sensitive compound (11) feeding the sterilizing device (27).

Certain embodiments of the present invention also relate to an apparatus wherein the heat treatment unit comprises:
- a direct heating device wherein the viscous matrix is heated up by incorporating steam into the viscous matrix,
- a high temperature circuitry, fed with the heated viscous matrix, wherein the heated viscous matrix is maintained in a temperature range of 120°C to 155°C for a predetermined residence time to allow sterilization of the viscous matrix, and
- an evaporative cooling device, fed with the sterilized viscous matrix, wherein the sterilized viscous matrix is cooled to a temperature of between 20°C to 90°C by steam evaporation.

In certain embodiments of the present invention, the direct heating unit comprises a steam infusion device. In certain embodiments, the direct heating unit comprises a steam injection device. Additionally, the apparatus may further comprise an indirect heating device located upstream the direct heating device, configured to feed the direct heating device with the viscous matrix at a temperature of between 20°C to 90°C.

In certain embodiments of the present invention, the heat treatment unit comprises:
- a first heat exchanger device wherein the viscous matrix is heated up,
- a high temperature circuitry, fed with the heated viscous matrix, wherein the heated viscous matrix is maintained in a temperature range of 120°C to 155°C for a predetermined residence time to allow sterilization of the viscous matrix, and
- a second heat exchanger, fed with the sterilized viscous matrix, wherein the sterilized viscous matrix is cooled to a temperature of between 20°C to 90°C.

In certain embodiments of the invention, the apparatus further comprises a sterilizing device located upstream the mixing unit, fed with the temperature sensitive compound.

In certain embodiments of the invention, the sterilizing device fed with the temperature sensitive compound comprises a filtration device or a heat treatment device. A device ensuring sterilization of the temperature sensitive compound by radiation exposure, irradiation exposure or ultra violet radiation is also considered by the present invention.

In certain embodiments of the invention, the apparatus further comprises a dosing pump located upstream the mixing unit, fed with the temperature sensitive compound and configured to dose the flow rate or quantity of temperature sensitive compound injected into the mixing unit. Additionally, the dosing pump may be located upstream the sterilizing device. The dosing pump may be a positive displacement pump.

It is another object of the present invention to propose a method for the preparation and sterilization of a viscous fluid using certain embodiments of the apparatus, and which comprises the steps of sterilizing the viscous matrix in the heat treatment device and incorporating the temperature sensitive compound into the sterilized matrix. In certain embodiments of the invention, the method further comprises a step of filling the viscous fluid in a sterile container. In certain embodiments of the invention, the method further comprises the steps of filling the viscous fluid in a non sterile container, and storing the container in a cold environment

There has thus been outlined, rather broadly, certain embodiments of the invention in order that the detailed description thereof herein may be better understood, and in order that the present contribution to the art may be better appreciated.

In this respect, before explaining at least one embodiment of the invention in detail, it is to be understood that the invention is not limited in its application to the details of construction and to the arrangements of the components set forth in the following description or illustrated in the drawings.

As such, those skilled in the art will appreciate that the conception upon which this disclosure is based may readily be utilized as a basis for the designing of other structures, methods and systems.

### BRIEF DESCRIPTION OF THE ACCOMPANYING DRAWINGS

Other features and advantages of the present invention will become clear from the following detailed description of the non limitative embodiments of the invention, referring to the accompanying Drawings representing schematically the apparatus of the invention. In the accompanying Drawings:
Fig. 1 is a flow diagram according to a first embodiment of an apparatus for the preparation and sterilization of a viscous fluid; and
Fig. 2 is a flow diagram according to a second embodiment of an apparatus for the preparation and sterilization of a viscous fluid; and
Fig. 3 is a flow diagram according to a third embodiment of an apparatus for the preparation and sterilization of a viscous fluid.

### DESCRIPTION OF PREFERRED EMBODIMENTS

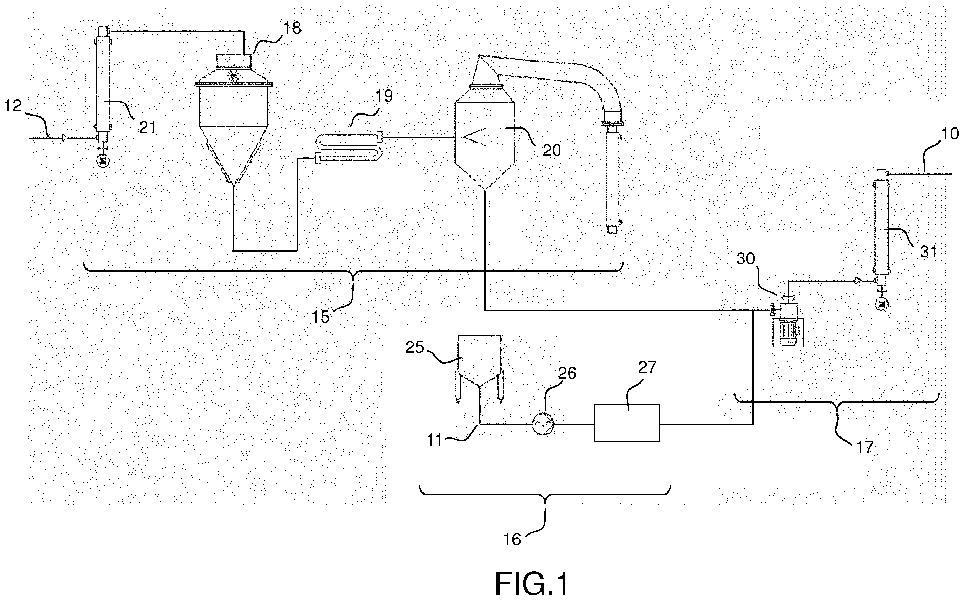

A flow diagram of a first embodiment of an apparatus for the preparation and sterilization of a viscous fluid is shown in detail in **Fig. 1****.** The apparatus is designed for cosmetic products, such as skincare creams or lotions, including emulsion products. However, the present invention is not limited to cosmetic products, but more generally covers, among other things, fluids of high viscosity containing a temperature sensitive active compound diluted in a viscous matrix. Other viscous fluids for which the present invention is adapted include, for example, drugs, vegetal and animal extracts, and food products. Examples of temperature sensitive active compounds include perfumes, essential oil, dyes, vitamins, proteins, pharmaceutical active compound or more generally active principals. A sterile temperature-sensitive compound is incorporated into a viscous matrix to form the viscous fluid. In the present application, a compound is considered sterile when it went through a sterilization process. Such compounds may be diluted in one or more types of viscous matrices, which may include for example an emulsion of oil into water or water into oil, a lipophilic medium, an aqueous or alcoholic medium or more generally any type of dilution substrate. Certain embodiments of the present invention aim at preparing and sterilizing any type of viscous fluids, preferably having a static viscosity exceeding 1500 cP at a temperature between approximately 20°C and 90°C. It has been shown that certain embodiments of the present invention are especially adapted to fluid for which a static viscosity exceeding 1500 cP is measured in this temperature range and after an evaporative cooling step, e.g. after sterilization based upon direct heating.

The flow diagram shown in Fig 1 describes the first embodiment of an apparatus for the preparation and sterilization of a viscous fluid 10, which contains at least one temperature sensitive active compound 11 diluted into a viscous matrix 12.

The apparatus comprises :
- a heat treatment unit 15, fed with the viscous matrix 12, wherein the viscous matrix is sterilized,
- a feeding unit 16 for the sterile temperature sensitive compound 11, and
- a mixing unit 17, fed with the sterilized viscous matrix 12 and the sterile temperature sensitive device 11, wherein the temperature sensitive compound is incorporated into the sterilized viscous matrix 12 to form the viscous fluid 10.

In the first embodiment, the heat treatment unit 15 includes a direct heating device 18 wherein the viscous matrix 12 is heated up by incorporating steam into the viscous matrix. The direct heating device 18 is a steam infusion device. It includes a chamber fed with steam, having an upper cylindrical part and a lower conical part. The inlet viscous matrix 12 is introduced in the upper cylindrical part and finely divided, for example by means of a dedicated perforated plate. Steam is incorporated into the matrix which is sprayed in the chamber. The lower conical part collects the heated matrix before transferring it to a high temperature circuitry 19. An efficient distribution of the steam flow, together with an efficient distribution of matrix inside the chamber, lead to an efficient heating of the viscous matrix. A temperature in the range of between approximately 120°C and 155°C may be reach in less than 1 second.

Subsequently, the heated viscous matrix circulates through a high temperature circuitry 19, wherein the heated viscous matrix is maintained in a temperature range of approximately 120°C to 155°C for a predetermined residence time to allow sterilization of the viscous matrix 12. As is known to the person of the art, the high temperature circuitry may include a holding tube and a combination of different kind of pumps or valves.

The heat treatment unit 15 also includes an evaporative cooling device 20 fed with the sterilized viscous matrix, wherein the viscous matrix is cooled to a temperature in the range of 20°C to 90°C by steam evaporation. In a possible embodiment, the evaporative cooling device 20 includes a vacuum chamber, having an upper cylindrical part and a lower conical part extending downwardly from the upper part. In the upper part is injected the heated viscous matrix 12. The matrix is then cooled down by evaporating, at least partially, the steam added to the fluid during the direct heating step. The lower part collects the cooled down matrix by gravitational force. Depending on the vacuum level and the matrix inlet and outlet temperatures, the amount of evaporated water can be adjusted. The pressure of vacuum chamber may be between approximately 0.05 and 1 bars (absolute pressure). A flash cooling duration of less than one second is achieved to cool down the fluid to a temperature of between approximately 20°C and 90°C. The temperature of the viscous matrix 12 at the outlet of the heat treatment unit 15 is set sufficiently low to ensure it does not denaturize the temperature sensitive compound 11 in the mixing unit 17. The cooled down matrix may be transported to the mixing unit 17 by direct pumping. But the high viscosity of the cooled down matrix at bottom of the lower part of vacuum chamber may make it difficult to extract the matrix out of the evaporative cooling device 20. For that matter, the evaporative cooling device may also include an extraction device, to extract the matrix from the lower part and ensure its transportation to the downstream circuit. As an example, the US patent application 13/713760 discloses an efficient design of an evaporative cooling device including such extraction device.

The heat treatment unit 15 may further include an indirect heating device 21 located upstream the direct heating device 18. The indirect heating device 21 aims at increasing the viscous matrix temperature up to an intermediate level to facilitate the introduction of the viscous matrix into the direct heating device 18 and the incorporation of steam. In a possible scenario, the indirect heating device 21, for example a heat exchanger, is configured to feed the direct heating device 18 with the viscous matrix at a temperature range of between approximately 20°C and 90°C.

As shown in Fig. 1, the feeding unit 16 of the temperature sensitive compound 11 may include a storage tank 25 and a dosing pump 26. The dosing pump 26 allows controlling the flow rate of temperature sensitive compound entering the mixing unit 17. In other words, the dosing pump 26 is configured to control the ratio of temperature sensitive compound to the viscous matrix. Many types of dosing pump may be used, depending on the properties of the temperature sensitive compound, the expected performance, etc... In a possible scenario, the dosing pump 26 is a positive displacement pump.

The feeding unit 16 may further include a sterilizing device 27 located upstream the mixing unit 17, fed with the temperature sensitive compound. The sterilizing device 27 may include a sterilizing filtration device, configured to filter largest undesired organic compounds. A device ensuring sterilization of the temperature sensitive compound by, for example, radiation exposure, irradiation exposure or ultra violet radiation is also considered by the present invention. The use of high pressure sterilizing device may also be used. The sterilizing device 27 may also include a heat treatment device such as a heat exchanger. In this case, the sterilizing device is configured to ensure some degree of sterilization of the temperature sensitive compound 11, while it prevents the compound from a significant denaturization that would occur at higher temperature. The temperature of the compound or its residence time throughout the sterilizing device 27 are kept at sufficiently low values, i.e. lower than the temperature or residence time values faced by the viscous matrix in the heat treatment unit 15.

In the embodiment as shown in Fig. 1, the sterilizing device 27 is located downstream the dosing pump 26. This arrangement allows the dosing pump to push the temperature sensitive compound through the sterilizing device 27, up to the mixing unit 17. This arrangement is also typically preferred to its alternative, wherein the pump is located downstream the sterilizing device, usually to avoid potential cavitation and/or to avoid the sterilized compound potentially passing through the pump.

The viscous fluid is prepared in the mixing unit 17 which combines the sterilized viscous matrix 12 and the temperature sensitive compound 11. In a possible embodiment, the mixing unit includes a dynamic mixer 30, for example comprising successive perforated plates and rotating blades inserted between the perforated plates. The viscous matrix and the temperature sensitive compound may be introduced separately into an agitated storage tank. Alternately, they may be combined before being introduced into the dynamic mixer. A possible solution is to configure the piping so that the temperature sensitive compound is introduced counter-current of the flow of viscous matrix. The resulting mixing is then introduced into the dynamic mixer to improve the homogenization of the mixing.

As shown on Fig. 1, the mixing unit 17 may further include a heat exchanger 31 to manage the temperature of the viscous fluid 10 at the outlet of the mixing unit 17. Optionally, the mixing unit may also include a heat exchanger at the inlet of the dynamic mixer 30 to manage the temperature of the viscous fluid in the mixer. To further improve the homogenization of the viscous fluid 10, the mixing unit 17 may include several successive dynamic mixers 30 and several heat exchangers 31 in between the mixers.

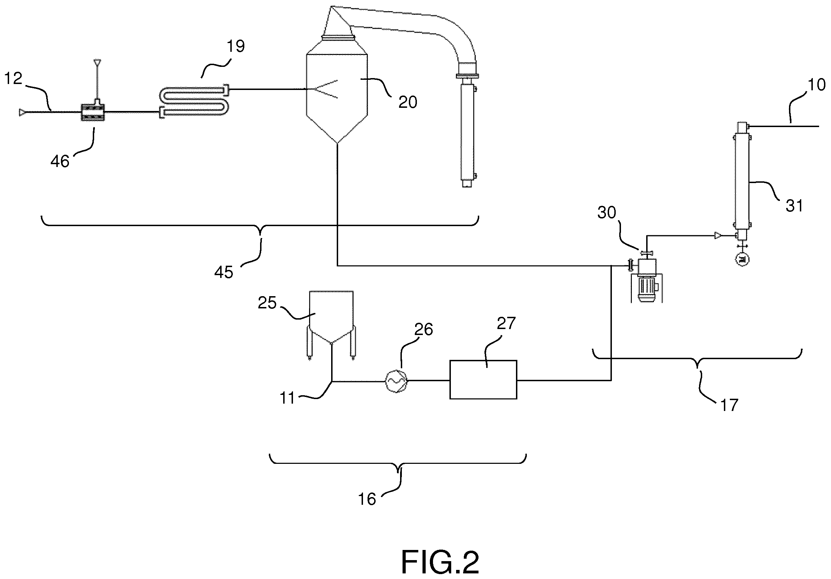

A flow diagram of a second embodiment of an apparatus for the preparation and sterilization of a viscous fluid is shown in detail in **Fig. 2****.** This second embodiment involves a number of components which have been described above in Fig. 1. The same numbers on the figures refer to the same components; these components are not detailed again systematically below.

The second embodiment includes a heat treatment unit 45 wherein the viscous matrix 12 is sterilized, a temperature sensitive compound feeding unit 16 and a mixing unit 17 wherein the viscous matrix 12 and the temperature sensitive compound 11 are mixed to form the viscous fluid 10. The designs of the feeding unit 16 and the mixing unit 17 are identical to the first embodiment. The second embodiment differs from the first one by the heat treatment unit. The heat treatment unit 45 of the second embodiment includes a direct heating device 46, wherein the viscous matrix 12 is heated up by incorporating steam into the viscous matrix, a high temperature circuitry 19, in which the viscous matrix circulates for a predetermined residence time to allow sterilizing the viscous matrix, and an evaporative cooling device 20, wherein the viscous matrix is cooled down by steam evaporation.

In this second embodiment, the direct heating device 46 is a steam injector device, though other direct heating devices are also within the scope of the present invention. The inlet viscous matrix 12, having a temperature in the range of between approximately 20°C and 90°C, for example thanks to an indirect preheating step (not represented on the figure), is brought to pass through a steam injection nozzle in which the viscous matrix is mixed with steam. The incorporation of steam heats up the fluid to a temperature in the range of between approximately 120°C and 155°C in less than 1 second. At the outlet of the direct heating device 46, the heated viscous matrix feeds the high temperature circuitry 19.

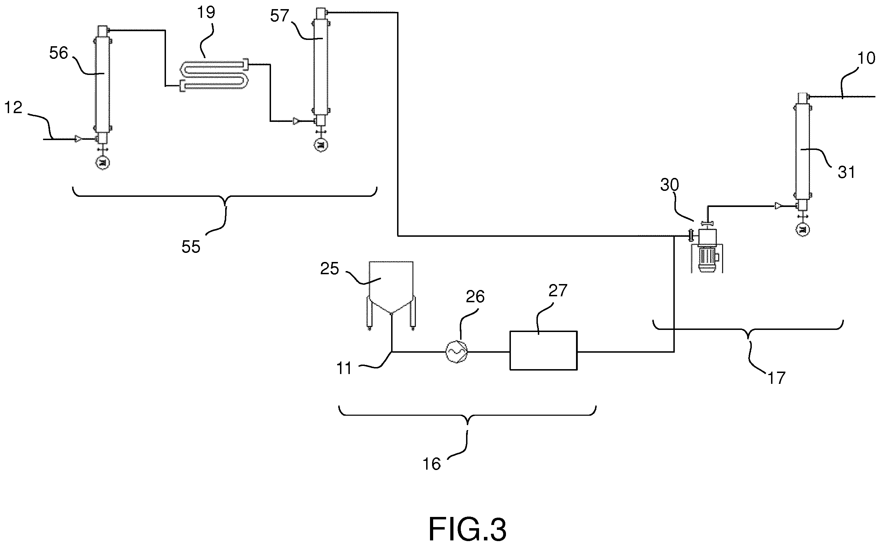

A flow diagram of a third embodiment of an apparatus for the preparation and sterilization of a viscous fluid is shown in detail in Fig. 3. This third embodiment involves a number of components which have been described above in Fig. 1 and Fig. 2. The same numbers on the figures refer to the same components; these components are not detailed again systematically below.

The third embodiment includes a heat treatment unit 55 wherein the viscous matrix 12 is sterilized, a temperature sensitive compound feeding unit 16 and a mixing unit 17 wherein the viscous matrix 12 and the temperature sensitive compound 11 are mixed to form the viscous fluid 10. The designs of the feeding unit 16 and the mixing unit 17 are identical to the first and second embodiment. The third embodiment differs from the previous ones by the heat treatment unit. The heat treatment unit 55 of the third embodiment is an indirect heating device. It includes a first heat exchanger 56 (or a first unit comprising several successive heat exchangers) to heat up the viscous matrix 12, a high temperature circuitry 19 to allow sterilizing the viscous matrix, and a second heat exchanger 57 (or a second unit comprising several successive heat exchangers) to cool down the sterilized viscous matrix 12.

Certain embodiment of the present invention also relates to a method for the preparation and sterilization of a viscous fluid containing a sterile temperature sensitive active compound diluted in a viscous matrix, which method comprises the successive steps of:
- sterilizing the viscous matrix in the heat treatment device, and
- incorporating the sterile temperature sensitive compound into the sterilized matrix.

## Claims

1. An apparatus for the preparation and sterilization of a viscous fluid (10) containing a sterile temperature sensitive compound (11) diluted into a viscous matrix (12), comprising :
- a heat treatment unit (15, 45, 55), fed with the viscous matrix (12), wherein the viscous matrix (12) is sterilized,
- a sterilizing device (27), fed with a temperature sensitive compound (11), ensuring the sterilization of the temperature sensitive compound (11),
- a mixing unit (17), fed with the sterilized viscous matrix (12) and the sterile temperature sensitive compound (11), wherein the temperature sensitive compound (11) is incorporated into the sterilized viscous matrix (12) to form the viscous fluid (10), the sterilizing device (27) being located upstream the mixing unit (17), **characterized in that** it further comprises :
- a dosing pump (26) located upstream the sterilizing device (27), fed with the temperature sensitive compound (11) and configured to dose the flow rate or quantity of temperature sensitive compound (11) feeding the sterilizing device (27).

2. The apparatus according to claim 1, wherein the heat treatment unit (15, 45) comprises:
- a direct heating device (18, 46) wherein the viscous matrix (12) is heated up by incorporating steam into the viscous matrix (12),
- a high temperature circuitry (19), fed with the heated viscous matrix (12), wherein the heated viscous matrix (12) is maintained in a temperature range of 120°C to 155°C for a predetermined residence time to allow sterilization of the viscous matrix (12), and
- an evaporative cooling device (20), fed with the sterilized viscous matrix (12), wherein the sterilized viscous matrix (12) is cooled to a temperature of between 20°C to 90°C by steam evaporation.

3. The apparatus according to claim 2, wherein the direct heating unit (15) comprises a steam infusion device (18).

4. The apparatus according to claim 2, wherein the direct heating unit (45) comprises a steam injection device (46).

5. The apparatus according to any of the claims 2 to 4, further comprising an indirect heating device (21) located upstream the direct heating device (18, 46), configured to feed the direct heating device (18, 46) with the viscous matrix (12) at a temperature of between 20°C to 90°C

6. The apparatus according to claim 1, wherein the heat treatment unit (55) comprises:
- a first heat exchanger device (56) wherein the viscous matrix (12) is heated up,
- a high temperature circuitry (19), fed with the heated viscous matrix (12), wherein the heated viscous matrix (12) is maintained in a temperature range of 120°C to 155°C for a predetermined residence time to allow sterilization of the viscous matrix (12), and
- a second heat exchanger (57), fed with the sterilized viscous matrix (12), wherein the sterilized viscous matrix (12) is cooled to a temperature of between 20°C to 90°C.

7. The apparatus according to claim 6, wherein the sterilizing device (27) fed with the temperature sensitive compound (11) comprises a filtration device or a heat treatment device, or a device ensuring sterilization by radiation exposure, irradiation exposure, or UV exposure, or a high pressure sterilizing device.

8. The apparatus according to any of the preceding claims, wherein the dosing pump (26) is a positive displacement pump.

9. The apparatus according to any of the claims 1 to 8, wherein the mixing unit (17) comprises a dynamic mixer (30) or an agitated storage tank.

10. A method for the preparation and sterilization of a viscous fluid (10) containing a sterile temperature sensitive compound (11) diluted into a viscous matrix (12), wherein the viscous fluid (10) circulates throughout an apparatus according to any of the claims 1 to 9, which method comprises the successive steps of:
- sterilizing the viscous matrix (12) in the heat treatment unit (15, 45, 55), and
- incorporating the sterile temperature sensitive compound (11) into the sterilized viscous matrix (12).

11. The method according to claim 10, further comprising a step of filling the viscous fluid in a sterile container.

## Patentansprüche

1. Vorrichtung zur Herstellung und Sterilisation eines viskosen Fluids (10), beinhaltend eine sterile, temperaturempfindliche Verbindung (11), die in eine viskose Matrix (12) verdünnt ist, umfassend:
- eine Wärmebehandlungseinheit (15, 45, 55), die mit der viskosen Matrix (12) gespeist wird, in dem die viskose Matrix (12) sterilisiert wird;
- ein Sterilisationsgerät (27), das mit einer temperaturempfindlichen Verbindung (11) gespeist wird, die die Sterilisation der temperaturempfindlichen Verbindung (11) gewährleistet;
- eine Mischereinheit (17), die mit der sterilisierten, viskosen Matrix (12) und der sterilen, temperaturempfindlichen Verbindung (11) gespeist wird, in der die temperaturempfindliche Verbindung (11) in die sterilisierte, viskose Matrix (12) aufgenommen wird, um das viskose Fluid (10) zu bilden, wobei sich das Sterilisationsgerät (27) stromaufwärts von der Mischereinheit (17) befindet,
**dadurch gekennzeichnet, dass** sie ferner umfasst:
- eine Dosierpumpe (26), die sich stromaufwärts von dem Sterilisationsgerät (27) befindet, die mit der temperaturempfindlichen Verbindung (11) gespeist wird und konfiguriert ist, die Durchflussrate oder -menge von temperaturempfindlicher Verbindung (11), die das Sterilisationsgerät (27) speist, zu dosieren.

2. Vorrichtung nach Anspruch 1, wobei die Wärmebehandlungseinheit (15, 45) Folgendes umfasst:
- ein direktes Heizgerät (18, 46), wobei die viskose Matrix (12) durch Aufnehmen von Dampf in die viskose Matrix (12) erhitzt wird;
- einen Hochtemperaturkreislauf (19), der mit der erhitzten, viskosen Matrix (12) gespeist wird, wobei die erhitzte, viskose Matrix (12) für eine vorbestimmte Verweilzeit in einem Temperaturbereich zwischen 120 °C und 155 °C gehalten wird, um eine Sterilisation der viskosen Matrix (12) zu ermöglichen; und
- ein Verdampfungskühlgerät (20), das mit der sterilisierten, viskosen Matrix (12) gespeist wird, wobei die sterilisierte, viskose Matrix (12) durch Verdampfung auf eine Temperatur zwischen 20 °C und 90 °C abgekühlt wird.

3. Vorrichtung nach Anspruch 2, wobei die direkte Heizungseinheit (15) ein Dampfinfusionsgerät (18) umfasst.

4. Vorrichtung nach Anspruch 2, wobei die direkte Heizungseinheit (45) ein Dampfeinspritzgerät (46) umfasst.

5. Vorrichtung nach einem der Ansprüche 2 bis 4, ferner umfassend ein indirektes Heizgerät (21), das sich stromaufwärts vor dem direkten Heizgerät (18, 46) befindet, das konfiguriert ist, um das direkte Heizgerät (18, 46) mit der viskosen Matrix (12) bei einer Temperatur zwischen 20 °C und 90 °C zu speisen.

6. Vorrichtung nach Anspruch 1, wobei die Wärmebehandlungseinheit (55) Folgendes umfasst:
- ein erstes Wärmetauschergerät (56), in dem die viskose Matrix (12) erhitzt wird;
- einen Hochtempertaturkreislauf (19), der mit der erhitzten, viskosen Matrix (12) gespeist wird, wobei die erhitzte, viskose Matrix (12) für eine vorbestimmte Verweilzeit in einem Temperaturbereich zwischen 120 °C und 155 °C gehalten wird, um eine Sterilisation der viskosen Matrix (12) zu ermöglichen; und
- einen zweiten Wärmetauscher (57), der mit der sterilisierten, viskosen Matrix (12) gespeist wird, in dem die sterilisierte, viskose Matrix (12) auf eine Temperatur zwischen 20 °C und 90 °C abgekühlt wird.

7. Vorrichtung nach Anspruch 6, wobei das Sterilisationsgerät (27), das mit der temperaturempfindlichen Verbindung (11) gespeist ist, ein Filtrationsgerät oder ein Wärmebehandlungsgerät, oder ein Gerät, das eine Sterilisation durch Strahlenaussetzung, Bestrahlungsaussetzung oder UV-Aussetzung gewährleistet, oder ein Hochdrucksterilisationsgerät umfasst.

8. Vorrichtung nach einem der vorhergehenden Ansprüche, wobei die Dosierpumpe (26) eine Verdrängerpumpe ist.

9. Vorrichtung nach einem der Ansprüche 1 bis 8, wobei die Mischereinheit (17) einen dynamischen Mischer (30) oder einen gerührten Speicherbehälter umfasst.

10. Verfahren zur Herstellung und Sterilisation eines viskosen Fluids (10), beinhaltend eine sterile, temperaturempfindliche Verbindung (11), die in eine viskose Matrix (12) verdünnt ist, wobei das viskose Fluid (10) durch eine Vorrichtung nach einem der Ansprüche 1 bis 9 zirkuliert, wobei das Verfahren die folgenden aufeinanderfolgenden Schritte umfasst:
- Sterilisation der viskosen Matrix (12) in der Wärmebehandlungseinheit (15, 45, 55), und
- Aufnehmen der sterilen, temperaturempfindlichen Verbindung (11) in die sterilisierte, viskose Matrix (12).

11. Verfahren nach Anspruch 10, ferner umfassend einen Schritt eines Füllens des viskosen Fluids in einen sterilen Behälter.

## Revendications

1. Appareil destiné à la préparation et à la stérilisation d'un fluide visqueux (10) contenant un composé stérile sensible à la température (11) dilué dans une matrice visqueuse (12), comprenant :
- une unité de traitement thermique (15, 45, 55), alimentée avec la matrice visqueuse (12), au sein duquel est stérilisée la matrice visqueuse (12),
- un dispositif de stérilisation (27), alimenté avec un composé sensible à la température (11), ce qui garantit la stérilisation du composé sensible à la température (11),
- une unité de mélange (17), alimentée avec la matrice visqueuse (12) stérilisée et le composé stérile sensible à la température (11), au sein de laquelle le composé sensible à la température (11) est incorporé dans la matrice visqueuse stérilisée (12) afin de former le fluide visqueux (10), le dispositif de stérilisation (27) se trouvant en amont de l'unité de mélange (17),
**caractérisé en ce qu'**il comprend en outre :
- une pompe de dosage (26) située en amont du dispositif de stérilisation (27), alimentée avec le composé sensible à la température (11) et configurée pour doser le débit ou la quantité de composé sensible à la température (11) alimentant le dispositif de stérilisation (27).

2. Appareil selon la revendication 1, dans lequel l'unité de traitement thermique (15, 45) comprend :
- un dispositif de chauffage direct (18, 46), au sein duquel la matrice visqueuse (12) est chauffée par incorporation de vapeur dans la matrice visqueuse (12),
- un circuit haute température (19), alimenté avec la matrice visqueuse (12) chauffée, au sein duquel la matrice visqueuse (12) chauffée est maintenue dans une plage de température comprise entre 120°C et 155°C pendant un temps de séjour prédéterminé afin de permettre une stérilisation de la matrice visqueuse (12), et
- un dispositif de refroidissement par évaporation (20), alimenté avec la matrice visqueuse (12) stérilisée, au sein duquel la matrice visqueuse (12) stérilisée est refroidie jusqu'à une température comprise entre 20°C et 90°C par évaporation de vapeur.

3. Appareil selon la revendication 2, dans lequel l'unité de chauffage direct (15) comprend un dispositif d'infusion de vapeur (18).

4. Appareil selon la revendication 2, dans lequel l'unité de chauffage direct (45) comprend un dispositif d'injection de vapeur (46).

5. Appareil selon l'une quelconque des revendications 2 à 4, comprenant en outre un dispositif de chauffage indirect (21) situé en amont du dispositif de chauffage direct (18, 46), configuré pour alimenter le dispositif de chauffage direct (18, 46) avec la matrice visqueuse (12) à une température comprise entre 20°C et 90°C.

6. Appareil selon la revendication 1, dans lequel l'unité de traitement thermique (55) comprend :
- un premier dispositif échangeur de chaleur (56), au sein duquel est chauffée la matrice visqueuse (12),
- un circuit haute température (19), alimenté avec la matrice visqueuse (12) chauffée, au sein duquel la matrice visqueuse (12) chauffée est maintenue dans une plage de température comprise entre 120°C et 155°C pendant un temps de séjour prédéterminé afin de permettre une stérilisation de la matrice visqueuse (12), et
- un deuxième échangeur de chaleur (57), alimenté avec la matrice visqueuse (12) stérilisée, au sein duquel la matrice visqueuse (12) stérilisée est refroidie jusqu'à une température comprise entre 20°C et 90°C.

7. Appareil selon la revendication 6, dans lequel le dispositif de stérilisation (27) alimenté avec le composé sensible à la température (11) comprend un dispositif de filtration ou un dispositif de traitement thermique, ou un dispositif garantissant une stérilisation grâce à une exposition à un rayonnement, une exposition à une irradiation, ou une exposition à des rayons UV, ou un dispositif de stérilisation haute pression.

8. Appareil selon l'une quelconque des revendications précédentes, dans lequel la pompe de dosage (26) est une pompe volumétrique.

9. Appareil selon l'une quelconque des revendications 1 à 8, dans lequel l'unité de mélange (17) comprend un mélangeur dynamique (30) ou un réservoir de stockage agité.

10. Procédé destiné à la préparation et à la stérilisation d'un fluide visqueux (10) contenant un composé stérile sensible à la température (11) dilué dans une matrice visqueuse (12), dans lequel le fluide visqueux (10) circule à travers un appareil selon l'une quelconque des revendications 1 à 9, ledit procédé comprenant les étapes successives consistant à :
- stériliser la matrice visqueuse (12) dans l'unité de traitement thermique (15, 45, 55), et
- incorporer le composé stérile sensible à la température (11) dans la matrice visqueuse (12) stérilisée.

11. Procédé selon la revendication 10, comprenant en outre une étape consistant à remplir un conteneur stérile avec le fluide visqueux.
